# EUROPEAN PATENT SPECIFICATION

(11) **EP 2 481 308 B1**
(45) Date of publication and mention of the grant of the patent: **29.04.2015**
(21) Application number: 10850020.8
(22) Date of filing: 12.06.2010
(51) Int. Cl.: A24F 47/00

(54) **COMBINED MULTIFUNCTIONAL ELECTRONIC SIMULATED CIGARETTE**
KOMBINIERTE ELEKTRONISCHE MEHRZWECK-SIMULATIONSZIGARETTE
CIGARETTE SIMULÉE ÉLECTRONIQUE MULTIFONCTIONNELLE COMBINÉE

(30) Priority: 22.04.2010 CN 201010153118
(43) Date of publication of application: 01.08.2012
(73) Proprietor: Xiu, Yunqiang, Qingdao, Shandong 266071 (CN)
(72) Inventor: Xiu, Yunqiang, Qingdao, Shandong 266071 (CN)
(74) Representative: Allen, Matthew Emmerson
(86) International application number: PCT/CN2010/000844
(87) International publication number: WO 2011/130886

(56) References cited:
- EP-A1- 0 488 488
- EP-A1- 1 736 065
- CN-A- 1 040 914
- CN-A- 101 268 867
- DE-C1- 4 328 243
- US-A- 4 694 824
- US-B2- 7 527 059

## Description

### Technical Field:

The invention claims a simulated cigarette, specifically a combined multifunctional electronic simulated cigarette.

### Background Art:

It is generally held by the public that smoking is harmful to human health; however, the nicotine in cigarette make smokers depend on cigarettes, which is one critical reason for smokers difficultly quitting smoking. Therefore, the simulated cigarette without nicotine, incapable of replacing the traditional cigarette, fails to meet the demands of smoking quitters at different stages. Additionally, some simulated cigarettes containing nicotine are likely to meet nicotine intake demand, but cannot reduce the dependence of smokers on nicotine while smoking the cigarette. Thus, it fails to assist smokers to quit smoking. Due to the above mentioned disadvantages of simulated cigarette, the electronic simulated cigarette, aiming at quitting smoking or replacing the present cigarette, has not been popularized yet.

An example of prior art disclosing a flavor delivering device is disclosed in EP 0 488 488 A1.

### Invention Description:

The combined multifunctional electronic simulated cigarette in the present invention can provide users with aromatic gases, nicotine component and simulated smoke simultaneously, and the users can respectively choose to take in the aromatic gases, the nicotine component or the simulated smoke according to the needs, and the invention can meet the needs of the users or the smoking quitters at different stages, help the users to gradually reduce nicotine intake amount, and finally achieve the purpose of not taking in nicotine.

For the above purpose, the invention is carried out through the following technical solution. A combined multifunctional electronic simulated cigarette comprising an indicator, the indicator is provided with a gas inlet hole; one end of the indicator is connected with one end of an aroma generator; a first switch and a first battery are arranged between the indicator and the aroma generator; a solid aromatic substance is arranged in the aroma generator and is provided with electric heating wires; the other end of the aroma generator is connected with one end of a smoke capsule, solid adsorption cotton adsorbed with nicotine is arranged in the smoke capsule; and the other end of the smoke capsule is connected with a suction nozzle on which a suction hole is provided; the smoke capsule is in detachable connection with the aroma generator and the suction nozzle. A rotary switch may be installed at one end of the indicator; an indicator lamp cover may be installed on the rotary switch; and a first indicator lamp and a second indicator lamp may be installed in the indicator lamp cover. The smoke capsule may be composed of a first smoke capsule shell and a second smoke capsule shell which are mutually connected, a first insert tube may be arranged in the first smoke capsule shell; a second insert tube may be arranged in the second smoke capsule shell ; the solid adsorption cotton may be installed in a cylindrical smoke capsule shell; and sealing aluminum foil films may be arranged at two ends of the cylindrical smoke capsule shell. A controller may be installed in the indicator; and a dynamic indicator lamp set may be fixed on the controller. The indicator may be of tobacco-pipe shape. The smoke capsule may be cone-shaped. Said solid aromatic substance may be composed of the following raw materials based on weight ratio: 15.5 wt.% of Mentha haplocalyx, 12 wt.% of fructus amomi, 12 wt.% of Syzygium aromaticum, 12 wt.% of cinnamon, 10 wt.% of rhizoma zingiberis, 10 wt.% of Pricklyash Peel, 12 wt.% of Foeniculum vulgare, 1.5 wt.% of vanillin, 12 wt.% of Ethyl Maltol, 1.5 wt.% of linalool extract, 0.5 wt.% of aromatic tobacco essential oil, 0.5 wt.% of Bulgaria rose oil, and 0.5 wt.% of Yunnan flue-cured tobacco essential oil. An atomization power source device may be arranged between the smoke capsule and the suction nozzle; a second switch and a power source may be installed in the atomization power source device; the suction nozzle may be provided with liquid adsorbing cotton containing atomized liquid; and the liquid adsorbing cotton may be provided with atomized heating wires which are connected with the atomization power source device. The atomized liquid in said liquid adsorbing cotton may be composed of raw materials based on the following weight ratio: 70-80 wt.% of glycerol and 20-30 wt.% of water..

The advantages of the invention are as follows: the invention can provide users with aromatic gases, the nicotine component and simulated smoke simultaneously; the users can respectively choose to take in the aromatic gases, the nicotine component and the simulated smoke according to the needs, and the invention can meet the needs of the users or the smoking quitters in different stages; it is featured with high simulation; the users are capable of showing whether the simulated cigarette in-service contains nicotine component or not through selecting the indicator lamps with different colors; moreover, it helps the users to gradually reduce nicotine intake amount, finally achieving the purpose of not taking in nicotine.

### Description of Figures:

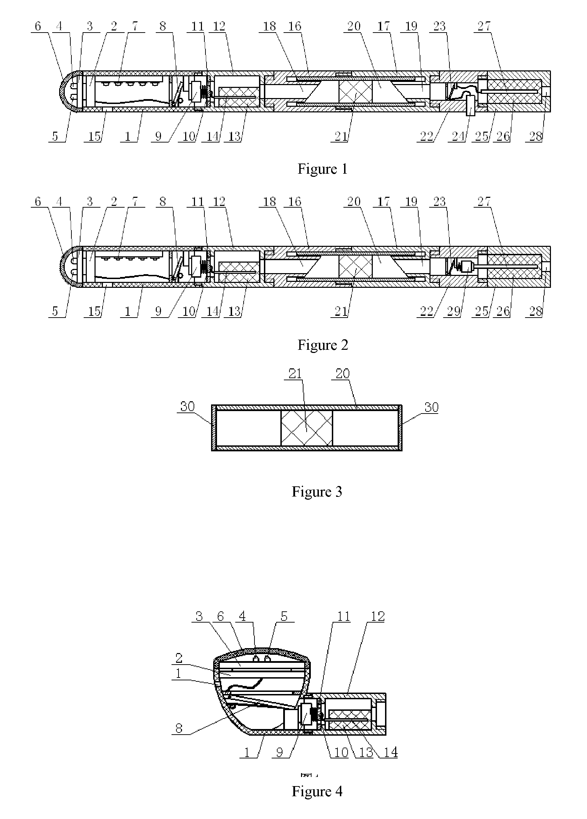

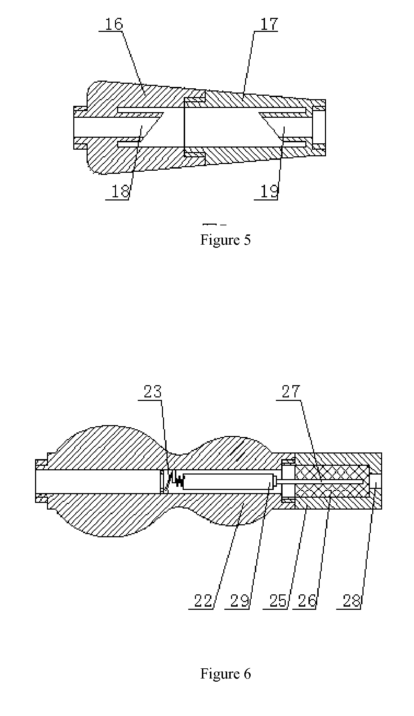

Fig. 1 shows the structure of the invention; Fig. 2 shows the structure of another example; Fig 3 shows the structure of smoke magazine in the invention; Fig. 4 shows the structure of the tobacco pipe-shaped indicator; Fig.5 shows the structure of the conical smoke capsule; and Fig. 6 shows the structure of the gourd-shaped atomization power source device.

### Description of the Preferred Embodiment:

A combined multifunctional electronic simulated cigarette comprising an indicator 1, the indicator 1 is provided with a gas inlet hole 15; one end of the indicator 1 is connected with one end of an aroma generator 12; a first switch 8 and a first battery 9 are arranged between the indicator 1 and the aroma generator 12; a solid aromatic substance 13 is arranged in the aroma generator 12 and is provided with electric heating wires 14; the other end of the aroma generator 12 is connected with one end of a smoke capsule, solid adsorption cotton 21 adsorbed with nicotine is arranged in the smoke capsule; and the other end of the smoke capsule is connected with a suction nozzle 25 on which a suction hole 28 is provided; the smoke capsule is in detachable connection with the aroma generator 12 and the suction nozzle 25. The gas enters the interior of simulated cigarette via the gas inlet hole 15 when the user inhales gases. When the gas passes through the first switch 8, the first switch 8 is capable of closing the power supply circuit of electric heating wires 14 to heat the solid aromatic substance 13. After the solid aromatic substance 13 is heated, the aromatic gas enters the smoke capsule along with the gas flow, which is mixed with nicotine substance in solid adsorption cotton 21 to form mixed gases. Finally, the mixed gases are sucked into the body of users through the suction hole 28 on the suction nozzle 25. Said first switch 8 can be a pneumatic switch which is started through gas flow, or a voice operated switch which is started through the sound while the gas passes through the switch. The smoke capsule is in detachable connection with the aroma generator 12 and the suction nozzle 25, such as threaded connection, trough connection or fastener connection. If the users are not required to suck nicotine, the smoke capsule can be dismantled, and the aroma generator 12 is connected the suction nozzle 25 to form the electronic simulated cigarette with aroma containing no nicotine component. The users are further capable of selecting to power on or power off the solid aromatic substance 13 according to their needs, thereby determining whether to take in aromatic gas.

The preferable structure of said indicator 1 is as follows: a rotary switch 3 is installed at one end of the indicator 1; an indicator lamp cover 6 is installed on the rotary switch 3; and a first indicator lamp 4 and a second indicator lamp 5 are installed in the indicator lamp cover 6, wherein the first indicator lamp 4 and the second indicator lamp 5 are of different colors; when the user sucks the gas, the first switch 8 is switched off to close the indicator lamp power supply circuit; when the indicator lamp lights on, and simultaneously the electric heating wires 14 in the solid aromatic substance 13 are warmed up to stimulate the aroma; and the user is capable of lighting on and lighting off the first indicator lamp 4 and the second indicator lamp 5 through the rotary switch 3. Moreover, the indicator lamp in the above structure is capable of being lighted on while the user sucks gas, or being lighted off. In addition, in comparison with the existing similar products, the simulated cigarette in the invention, with higher simulation, is more similar to the real cigarette. Meanwhile, the structure is further advantaged in the capability of indicating the current operation status thereof. In other words, the user is capable of selecting to light up indicator lamps with different colors to show whether the simulated cigarette in-service contains nicotine component. The rotary switch 3 in the above technical solution can be replaced by a push switch. Of course, said indicator 1 in the invention can be of other structures. For instance, the rotary switch 3 in the preferable technical solution is not a necessity, and only utilizing one indicator lamp is acceptable. This technical solution is also provided with relatively high simulation. However, the user is not capable of selecting to light up indicator lamps of different colors to show whether the simulated cigarette in-service contains nicotine component.

For the convenience of replacing the solid adsorption cotton 21 in said smoke capsule, the invention can use the structure as follows: the smoke capsule is composed of a first smoke capsule shell 16 and a second smoke capsule shell 17 which are mutually connected; a first insert tube 18 is arranged in the first smoke capsule shell 16; a second insert tube 19 is arranged in the second smoke capsule shell 17; the solid adsorption cotton 21 is installed in a cylindrical smoke capsule shell 20; and sealing aluminum foil films 30 are arranged at two ends of the cylindrical smoke capsule shell 20. Once required to replace the solid adsorption cotton 21, the smoke capsule shell 20 can be taken out after separating the first smoke capsule shell 16 and the second smoke capsule shell 17. Furthermore, the smoke capsule shell 20 and the sealing aluminum foil films 30 play a role in preventing the liquid evaporation in the solid adsorption cotton 21 for storage. The first insert tube 18 and the second insert tube 19 are applied to pierce the sealing aluminum foil films 30.

For further improvement in simulation, a controller 2 can be installed in the indicator 1; and a dynamic indicator lamp set 7 is fixed on the controller 2. Lamp set 7 composed of multiple transversely arranged indicator lamps. The first battery 9 supplies power for it. When the user inhales gas, the dynamic indicator lamp set 7 is powered off and the indicator lamps are lighted on and off under the control of the controller 2 to simulate the gradually burned out cigarette while smoking. With the above technical solution, the shell of said indicator 1 is made of a transparent material, or an opaque groove can be opened on a nontransparent material.

In order to meet the demands of users at different levels, the indicator 1 can be of tobacco-pipe shape. On top of meeting the needs of routine smoking, said simulated cigarette in the present invention can further be sold as handicraft articles. Additionally, said indicator 1 can also be of cylindrical or else. The cylindrical simulated cigarette is similar to the ordinary cigarette, in concordance with the habit of users.

The appearance of said smoke capsule can be made into various shapes as required, e.g. conical or cylindrical. The conical cigarette, with antiskid effect, is more suitable with the posture of the user holding the cigarette, and the user is more comfortable while using. While the overall appearance of the cylindrical simulated cigarette is identical to that of the ordinary cigarette, in concordance with the habit of users.

The solid aromatic substance 13 can be composed of the following raw materials based on weight ratio. The preferable weight ratio of the raw material is as follows: 15.5 wt.% of Mentha haplocalyx, 12 wt.% of fructus amomi, 12 wt.% of Syzygium aromaticum, 12 wt.% of cinnamon, 10 wt.% of rhizoma zingiberis, 10 wt.% of Pricklyash Peel, 12 wt.% of Foeniculum vulgare, 1.5 wt.% of vanillin, 12 wt.% of Ethyl Maltol, 1.5 wt.% of linalool extract, 0.5 wt.% of aromatic tobacco essential oil, 0.5 wt.% of Bulgaria rose oil, and 0.5 wt.% of Yunnan flue-cured tobacco essential oil. The raw material consists of multiple Chinese herbal medicines and essential oil so that the solid aromatic substance 13 is advantaged in pure aroma and easy volatilization after being heated. Furthermore, some Chinese herbal medicines play a role in health care.

To get better smoke of the simulated cigarette, an atomization power source device 22 can be arranged between the smoke capsule and the suction nozzle 25; a second switch 23 and a power source are installed in the atomization power source device 22; the suction nozzle 25 is provided with liquid adsorbing cotton 26 containing atomized liquid; the liquid adsorption cotton 26 is adsorbed with atomized liquid composed of glycerol and water to make the atomized liquid generate atomization effect after being heated and expanded; and the liquid adsorbing cotton 26 is provided with atomized heating wires 27 which are connected with the atomization power source device 22. A second cell 29 can be arranged to directly supply power for the atomization power source device 22 in direct current. Alternatively, a mobile phone charging interface 24 can be electrically connected with external mobile phone power source to supply power for the atomized heating wires 27. When the user inhales gas, the gas flow passes through the second switch 23, and power supply circuit of atomized heating wires 27 can be switched off via the second switch 23; after powering on the atomized heating wires 27, the liquid adsorption cotton 26 is heated to make the atomized liquid generate excellent atomization effect, achieving the smoke effect of ordinary cigarette. The second switch 23 can be a pneumatic switch which is started through gas flow or a voice operated switch which is started through the sound while the gas passes through the switch.

Said atomized liquid is composed of raw materials based on the following weight ratio: 70-80wt.% of glycerol and 20-30 wt.% of water. The combination of glycerol and water is capable of making the atomized liquid generate a lot of smoke to simulate the smoke effect of ordinary cigarette. The mixture of water and glycerol is not harmful to human health, without influencing the taste of simulated cigarette. The atomized liquid in said liquid adsorbing cotton 26 is composed of raw materials based on the following weight ratio:
1. 70 wt.% of glycerol and 30 wt.% of water;
2. 80 wt.% of glycerol and 20 wt.% of water;
3. 75 wt.% of glycerol and 25 wt.% of water.

When the smoke capsule is dismantled by the user for smoking, 0.6-2.4 wt.% of nicotine component can be added into the raw materials of atomized liquid to meet nicotine intake needs of those who hold a craving for tobacco, to the benefit of gradually reducing nicotine intake amount, finally achieving the purpose of not taking in nicotine. The weight ratio of nicotine added in the raw materials of atomized liquid is as follows:
1. 70 wt.% of glycerol, 29.4 wt. % of water and 0.6 wt. % of nicotine;
2. 70 wt.% of glycerol, 27.6 wt. % of water and 2.4 wt. % of nicotine;
3. 70 wt.% of glycerol, 28.5 wt. % of water and 1.5 wt. % of nicotine;
4. 69.4 wt.% of glycerol, 30 wt. % of water and 0.6 wt. % of nicotine;
5. 67.6 wt.% of glycerol, 30 wt. % of water and 2.4 wt. % of nicotine;
6. 68.5 wt.% of glycerol, 30 wt. % of water and 1.5 wt. % of nicotine;
7. 80 wt.% of glycerol, 19.4 wt. % of water and 0.6 wt. % of nicotine;
8. 78.8 wt.% of glycerol, 18.8 wt. % of water and 2.4 wt. % of nicotine;
9. 18.5 wt.% of glycerol, 20 wt. % of water and 1.5 wt. % of nicotine.

Said atomization power source device 22 can be cylindrical, gourd-shaped or else. The cylindrical simulated cigarette is identical to the ordinary cigarette, in concordance with the habit of users. The gourd-shaped simulated cigarette is capable of serving as an ornamental article and assisting the user to find the pipe end under dark conditions. If not taking in the simulated smoke, the user can power off the atomization power source device 22 to stop heating the atomized liquid through the atomized heating wires 27. At this moment, the gases getting out from the suction hole 28 are not atomized. In the figure, the component 10 is a clapboard and the component 11 is a vent hole.

## Claims

1. A combined multifunctional electronic simulated cigarette comprising an indicator (1), wherein the indicator (1) is provided with a gas inlet hole (15); one end of the indicator (1) is connected with one end of an aroma generator (12); a first switch (8) and a first battery (9) are arranged between the indicator (1) and the aroma generator (12); a solid aromatic substance (13) is arranged in the aroma generator (12) and is provided with electric heating wires (14); the other end of the aroma generator (12) is connected with one end of a smoke capsule, solid adsorption cotton (21) adsorbed with nicotine is arranged in the smoke capsule; and the other end of the smoke capsule is connected with a suction nozzle (25) on which a suction hole (28) is provided; the smoke capsule is in detachable connection with the aroma generator (12) and the suction nozzle (25).

2. The combined multifunctional electronic simulated cigarette according to Claim 1, wherein a rotary switch (3) is installed at one end of the indicator (1); an indicator lamp cover (6) is installed on the rotary switch (3); and a first indicator lamp (4) and a second indicator lamp (5) are installed in the indicator lamp cover (6) .

3. The combined multifunctional electronic simulated cigarette according to Claim 1, wherein the smoke capsule is composed of a first smoke capsule shell (16) and a second smoke capsule shell (17) which are mutually connected; a first insert tube (18) is arranged in the first smoke capsule shell (16); a second insert tube (19) is arranged in the second smoke capsule shell (17); the solid adsorption cotton (21) is installed in a cylindrical smoke capsule shell (20); and sealing aluminum foil films (30) are arranged at two ends of the cylindrical smoke capsule shell (20).

4. The combined multifunctional electronic simulated cigarette according to Claim 1 or 2, wherein a controller (2) is installed in the indicator (1); and a dynamic indicator lamp set (7) is fixed on the controller (2).

5. The combined multifunctional electronic simulated cigarette according to Claim 1 or 2, wherein the indicator (1) is of tobacco-pipe shape.

6. The combined multifunctional electronic simulated cigarette according to Claim 1 or 3, wherein the smoke capsule is cone-shaped.

7. The combined multifunctional electronic simulated cigarette according to Claim 1, wherein said solid aromatic substance (13) is composed of the following raw materials based on weight ratio: 15.5 wt.% of Mentha haplocalyx, 12 wt.% of fructus amomi, 12 wt.% of Syzygium aromaticum, 12 wt.% of cinnamon, 10 wt.% of rhizoma zingiberis, 10 wt.% of Pricklyash Peel, 12 wt.% of Foeniculum vulgare, 1.5 wt.% of vanillin, 12 wt.% of Ethyl Maltol, 1.5 wt.% of linalool extract, 0.5 wt.% of aromatic tobacco essential oil, 0.5 wt.% of Bulgaria rose oil, and 0.5 wt.% of Yunnan flue-cured tobacco essential oil.

8. The combined multifunctional electronic simulated cigarette, according to Claim 1 or 3, wherein an atomization power source device (22) is arranged between the smoke capsule and the suction nozzle (25); a second switch (23) and a power source are installed in the atomization power source device (22); the suction nozzle (25) is provided with liquid adsorbing cotton (26) containing atomized liquid; and the liquid adsorbing cotton (26) is provided with atomized heating wires (27) which are connected with the atomization power source device (22).

9. The combined multifunctional electronic simulated cigarette according to Claim 8, wherein the atomized liquid in said liquid adsorbing cotton (26) is composed of raw materials based on the following weight ratio: 70-80 wt.% of glycerol and 20-30 wt.% of water.

## Patentansprüche

1. Eine kombinierte, multifunktionale E-Zigarette mit einer Anzeige (1), wobei die Anzeige (1) mit einem Loch für den Gaseinlaß (15) versehen ist; ein Ende der Anzeige (1) ist mit dem anderen Ende des Aromagenerators (12) verbunden; der erste Schalter (8) und die erste Batterie (9) sind zwischen der Anzeige (1) und dem Aromagenerator (12) angeordnet; eine feste aromatische Substanz (13) ist in dem Aromagenerator (12) angebracht und ist mit den elektrischen Heizdrähten (14) ausgestattet; das andere Ende des Aromagenerators (12) ist mit einem Ende der Rauchkapsel verbunden; die feste Adsorptionsbaumwolle (21) ist in der Rauchkapsel angeordnet und adsorbiert das Nikotin, das andere Ende der Rauchkapsel ist mit einer Saugdüse (25) verbunden, auf dem ein Ansaugloch (28) eingepasst ist; die Rauchkapsel ist verbunden mit dem Aromagenerator (12) und der Saugdüse (25), jedoch abnehmbar.

2. Die kombinierte, multifunktionale E-Zigarette gemäß Anmerkung 1, darin befindet sich ein Drehscnalter (3), der an einem Ende der Anzeige (1) installiert ist; eine Abdeckung der Anzeigelampe (6) ist am Drehschalter (3) installiert; die erste Anzeigelampe (4) und die zweite Anzeigelampe (5) sind an der Anzeigelampenabdeckung (6) installiert.

3. Die kombinierte, multifunktionale E-Zigarettenach gemäß Anmerkung 1, darin besteht die Rauchpatrone aus der ersten Rauchkapselhülle (16) und der zweiten Rauchkapselhülle (17), die miteinander verbunden sind; das erste Einsatzrohr (18) ist in der Rauchkapselhülle (16) angeordnet; das zweite Einsatzrohr (19) ist in der zweiten Rauchkapselhülle (17) angeordnet; die feste Adsorptionsbaumwolle (21) ist in einer zylindrischen Rauchkapselhülle (20) installiert; Dichtungsfolie aus Aluminium (30) ist an beiden Enden der zylindrischen Rauchkapselhülle (20) angebracht.

4. Die kombinierte, multifunktionale E-Zigarette gemäß Anmerkung 1 oder 2, darin ist die Steuereinheit (2) in der Anzeige (1) installiert; ein Satz dynamischer Anzeigelampen (7) ist an der Steuereinheit (2) befestigt.

5. Die kombinierte, multifunktionale E-Zigarette gemäß Anmerkung 1 oder 2, darin ist die Anzeige (1) in Form des Tabakrohrs.

6. Die kombinierte, multifunktionale E-Zigarette gemäß Anmerkung 1 oder 3, darin ist die Rauchpatrone kegelförmig.

7. Die kombinierte, multifunktionale E-Zigarette gemäß Anmerkung 1, darin setzt sich die feste aromatische Substanz (13) aus folgenden Rohstoffen zusammen, basierend auf dem Gewicht: 15,5 % Gew. Feldminze, 12 % Gew. Amomumfrüchte, 12 % Gew. Gewürznelke, 12 % Gew. Zimt, 10 % Gew. Ingwerwurzelstock, 10 % Gew. Rinde der Stachelesche, 12 % Gew. Fenchel, 1,5 % Gew. Vanillin, 12 % Gew. Ethylmaltol, 1,5 % Gew. Linaloolextrakt, 0,5 % Gew. ätherisches Öl aromatischen Tabaks, 0,5 % Gew. Bulgarisches Rosenöl und 0,5 % Gew. ätherisches Öl heißluftgetrockneten Yunnan-Tabaks.

8. Die kombinierte, multifunktionale E-Zigarette gemäß Anmerkung 1 oder 3, darin ist das Energiequellengerät für die Zerstäubung (22) zwischen der Rauchkapsel und der Saugdüse (25) angeordnet; der zweite Schalter (23) und die Stromquelle sind in dem Energiequellengerät für die Zerstäubung (22) installiert; die Saugdüse (25) wird mit flüssigkeitsadsorbierender Baumwolle (26) bereitgestellt, welches die zerstäubte Flüssigkeit auffängt; die flüssigkeitsadsorbierende Baumwolle (26) ist mit Heizdrähten für die Zerstäubung (27) ausgestattet, die mit dem Energiequellengerät für Zerstäubung (22) verbunden sind.

9. Die kombinierte, multifunktionale E-Zigarette gemäß Anmerkung 8, darin besteht die zerstäbte Flüssigkeit in der benannten flüssigkeitsadsorbierenden Baumwolle (26) aus Rohstoffen auf Basis des folgendem Gewichtsverhältnisses: 70-80 % Gew. Glycerin und 20-30 % Gcw. Wasser.

## Revendications

1. Une cigarette électronique de simulation multifonctionnelle combinée comprenant un indicateur (1), dans lequel l'indicateur (1) est équipé d'un trou d'entrée de gaz (15); une extrémité de l'indicateur (1) est connectée avec une extrémité du générateur d'arôme (12); un premier bouton (8) et une première batterie (9) sont arrangés entre l'indicateur (1) et le générateur d'arôme (12); une substance aromatique solide (13) est disposée dans le générateur d'arôme (12) et est équipée de fils de chauffage électrique (14); l'autre extrémité du générateur d'arôme (12) est connecté avec une extrémité de la capsule de fumée, le coton d'absorption solide (21) absorbe la nicotine et l'arrange dans la capsule de fumée; et l'autre extrémité de la capsule de fumée est connectée avec une buse de succion (25) sur laquelle un trou de succion (28) est équipé; la capsule de fumée est connectée mais détachable du générateur d'arôme (12) et de la buse de succion (25).

2. La cigarette électronique de simulation multifonctionnelle combinée correspondant à la réclamation 1, sur laquelle un bouton rotatif (3) est installé à une extrémité de l'indicateur (1); un bouton lumineux de protection (6) est installé sur le bouton rotatif (3); et un premier indicateur lumineux (4) et un second indicateur lumineux (5) sont installés sur le bouton lumineux de protection (6).

3. La cigarette électronique de simulation multifonctionnelle combinée conformément à la réclamation 1, dans laquelle la capsule de fumée est composée d'une première coque de capsule de fumée (16) et d'une seconde coque de capsule de fumée (17) qui sont mutuellement connectées; un premier tube d'insert (18) est arrangé dans la première capsule de fumée (16); un deuxième tube d'insert (19) est installé dans la seconde capsule de fumée (17) ;le coton d'absorption solide (21) est installé dans coque de capsule de fumée cylindrique(20); et des films aluminium d'étanchéité (30) sont disposée aux deux extrémités de la coque de capsule de fumée (20).

4. La cigarette électronique de simulation multifonctionnelle combinée conformément à la réclamation 1 ou 2, sur laquelle un contrôleur (2) est installé dans l'indicateur (1); et un jeu de lampe dynamique (7) est fixé sur le contrôleur (2).

5. La cigarette électronique de simulation multifonctionnelle combinée conformément à la réclamation 1 ou 2, sur laquelle l'indicateur (1) est en forme de pipe à tabac.

6. La cigarette électronique de simulation multifonctionnelle combinée conformément à la réclamation 1 ou 3, sur laquelle la capsule de fumée est en forme de cône.

7. La cigarette électronique de simulation multifonctionnelle combinée conformément à la réclamation 1, dans laquelle ladite substance aromatique (13) est composée des matières premières suivantes en fonction du ratio du poids: 15,5% du poids de Mentha haplocalyx, 12% du poids de fructus amomi, 12% du poids de Sysygium aromaticum, 12%) du poids de cannelle, 10% du poids de rhizoma zingiberis, 10% du poids de Pricklyash Peel, 12% du poids de Foeniculum vulgarc, 1,5% du poids de vanilline, 12% du poids d'Ethyl Maltol, 1,5% du poids d'extrait de linalool, 0,5% du poids d'huile essentielle de tabac aromatique, 0,5% du poids d'huile de rose de bulgarie et 0,5% du poids d'huile essentielle de tabac flue-cured du Yunnan.

8. La cigarette électronique de simulation multifonctionnelle combinée, conformément à la réclamation 1 ou 3, sur laquelle un dispositif d'alimentation d'atomisation (22) est installé entre la capsule de fumée et la buse de succion (25); un deuxième bouton (23) et une source d'alimentation sont installés dans le dispositif d'alimentation d'atomisation (22); la buse de succion (25) est équipée d'un coton d'absorption de liquide (26) contenant le liquide à atomiser; et le coton d'absorption du liquide; et le coton d'absorption du liquide (26) est équipé de fils de chauffage d'atomisation (27) qui sont connectés sur le dispositif d'alimentation d'atomisation (22).

9. La cigarette électronique de simulation multifonctionnelle combinée conformément à la réclamation 8, dans laquelle le liquide atomisé dans ledit coton d'absorption de liquide (26) est composé de matières premières sur la base du ratio de poids suivant : 70-80% du poids de glycerol et 20-30% d'eau.
